# EUROPEAN PATENT APPLICATION

(11) **EP 3 974 126 A1**
(43) Date of publication of application: **30.03.2022**
(21) Application number: 20197720.4
(22) Date of filing: 23.09.2020
(51) Int. Cl.: B26B 19/38, B26B 21/40

(54) **INTERACTING WITH A USER OF A PERSONAL CARE DEVICE**

(71) Applicant: Koninklijke Philips N.V., 5656 AG Eindhoven (NL)
(72) Inventor: WANG, Lu, 5656 AE Eindhoven (NL); Bourquin, Yannyk Parulian Julian, 5656 AE Eindhoven (NL); Eren, Mustafa Tolga, 5656 AE Eindhoven (NL); Varghese, Babu, 5656 AE Eindhoven (NL); Palero, Jonathan Alambra, 5656 AE Eindhoven (NL)
(74) Representative: Philips Intellectual Property & Standards

(57) **Abstract**

According to an aspect, there is provided a computer-implemented method (300; Fig. 3) for interacting with a user of a personal care device (Fig. 1; 102). The method (300; Fig. 3) comprises receiving (302, Fig. 3) device usage data indicative of a manner in which the personal care device is used by the user during a usage instance of the personal care device, the device usage data relating to one or more of a defined set of device usage parameters; receiving (304) performance data indicative of an outcome of the usage instance, the performance data relating to one or more of a defined set of performance parameters; and responsive to a determination that one or more of the received device usage data and the received performance data differ from stored reference device usage data and stored reference performance data, respectively, by more than a defined threshold amount, generating (306) a user input request to be presented to the user. An apparatus, a system and a computer program product are also provided.

## Description

### FIELD OF THE INVENTION

The invention relates to personal care devices and, in particular, to interacting with a user of a personal care device.

### BACKGROUND OF THE INVENTION

Personal care devices may be used to perform personal care activities, such as shaving, hair trimming, tooth brushing, flossing, and the like. Some personal care devices may measure data while a personal care activity is being performed, and a user of the personal care device may be provided with feedback relating to the personal care activity, such as feedback informing the user that they are not brushing their teeth for long enough.

Determinations and measurements made in respect of a personal care activity may be inaccurate, may become less accurate over time, or may not take into account relevant information, such as a change that has taken place in respect of the user that is relevant to the personal care activity. Therefore, feedback provided to the user may not be as accurate as possible.

There is a desire, therefore, to provide a mechanism by which feedback to be provided to the user may be improved.

### SUMMARY OF THE INVENTION

One way of improving feedback to be provided to a user of a personal care device is to obtain input from the user. The inventors of the present disclosure have recognised that information provided by the user of a personal care device can be used to improve the feedback provided to the user in respect of their use of the device and may, in some cases, be used to tailor the feedback to a particular user. Moreover, the inventors have recognised that, by requesting the user input only on particular occasions, at appropriate times, the user experience is improved as the user does not become burdened with providing input repeatedly. Input provided by the user may be used to improve the accuracy of any determinations made in respect of the use of the personal care device, such that future feedback provided to the user is more accurate and the user is asked to provide their input less often.

According to a first specific aspect, there is provided a computer-implemented method for interacting with a user of a personal care device, the method comprising receiving device usage data indicative of a manner in which the personal care device is used by the user during a usage instance of the personal care device, the device usage data relating to one or more of a defined set of device usage parameters; receiving performance data indicative of an outcome of the usage instance, the performance data relating to one or more of a defined set of performance parameters; and responsive to a determination that one or more of the received device usage data and the received performance data differ from stored reference device usage data and stored reference performance data, respectively, by more than a defined threshold amount, generating a user input request to be presented to the user.

In this way, the user is asked to provide input only when it is necessary, for example to clarify or confirm data measured in respect of a usage instance, or to make adjustments to parameters of a model used to analyse the measured data.

In some embodiments, the method may comprise receiving a user input relating to the usage instance of the personal care device; and adjusting the defined threshold amount according to the received user input.

The user input request may comprise a request for confirmation of one or more of the received device usage data and the received performance data. In other embodiments, the user input request may comprise a request for an indication of the user's perceived outcome of the usage instance relating to one or more of a defined set of perceived performance parameters.

The personal care device may, in some embodiments, comprise a shaving device. Each perceived performance parameter may comprise an outcome parameter selected from a group comprising: a perceived closeness of shave achieved using the shaving device; an indication of any perceived skin irritation resulting from the usage instance; and an indication of perceived comfort following the usage instance.

Each device usage parameter may comprise a usage parameter selected from a group comprising: a motion of the personal care device; a pressure applied by the personal care device onto a surface of the body of the user; a speed of movement of the personal care device; and a direction of motion of the personal care device.

In embodiments where the personal care device comprises a shaving device, each outcome parameter may comprise an outcome parameter selected from a group comprising: a closeness of shave achieved using the shaving device; an indication of skin irritation resulting from the usage instance; and an indication of redness of skin resulting from the usage instance.

In some embodiments, the method may further comprise receiving context data indicative of the environment of the user of the personal care device during the usage instance; and generating a user input request responsive to determining that the context data differs from stored reference context data by more than a defined threshold amount.

The method may, in some embodiments, comprise, responsive to a determination that one or more of the received device usage data and the received performance data differ from stored reference device usage data and stored reference performance data, respectively, by more than a defined threshold amount, in respect of two or more device usage parameters or performance parameters, generating a user input request to be presented to the user, the user input request being in respect of the device usage parameter or performance parameter corresponding to the largest determined difference.

In some embodiments, at least one of the stored reference device usage data and the stored reference performance data may comprise data relating to at least one previous device usage instance of the personal care device by the user.

According to a second specific aspect, there is provided an apparatus for interacting with a user of a personal care device. The apparatus comprises a processor configured to perform steps of the methods disclosed herein.

In some embodiments, the apparatus may further comprise a storage device. The processor may be configured to store one or more of the received device usage data and the received performance data in the storage device.

The apparatus may further comprise one or more sensors configured to acquire the device usage data and/or the performance data.

According to a third specific aspect, there is provided a system comprising one or more sensors for acquiring device usage data indicative of a manner in which a personal care device is used by a user during a usage instance of the personal care device; and performance data indicative of an outcome of the usage instance. The system further comprises a storage module for storing reference device usage data and reference performance data; a comparison module for comparing the acquired device usage data with the stored reference device usage data and for comparing the acquired performance data with the stored performance data; and a user interface for presenting a user input request responsive to a determination that: the acquired device usage data differs from the stored reference device usage data by more than a first defined threshold amount; or the acquired performance data differs from the stored reference performance data by more than a second defined threshold amount.

According to a fourth specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform one or more steps of the methods disclosed herein.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of an example of a system for interacting with a user of a personal care device;
Fig. 2 is a schematic illustration of an example of an apparatus for interacting with a user of a personal care device;
Fig. 3 is a flowchart of an example of a method for interacting with a user of a personal care device;
Fig. 4 is a flowchart of a further example of a method for interacting with a user of a personal care device; and
Fig. 5 is a schematic illustration of an example of a processor in communication with a computer-readable medium.

### DETAILED DESCRIPTION OF THE EMBODIMENTS

According to various embodiments of the present disclosure, a mechanism is provided by which a user of a personal care device may be prompted to provide input at an appropriate time (e.g. at a time that is convenient for the user), which can help to improve feedback provided to the user regarding the personal care device and/or its use. In some embodiments of the invention, data is acquired regarding the use of the personal care device, which may, for example, indicate how the user is using the personal care device during a personal care activity. Data is also acquired regarding an outcome of the personal care activity, for example data indicative of the results of the personal care activity. Both the data regarding the use of the personal care device and the data regarding the outcome of the personal care activity are compared to respective reference data and, if either varies from the respective reference data by more than a defined amount, then the user of the personal care device may be prompted to provide input. In this way, the user can be asked for input if the use of the device is not as expected or if an outcome of the personal care activity is not as expected. In general, embodiments of the invention determine when to ask a user to provide input, and what input to request from the user.

In a specific example, a person may use a shaving device to shave their facial hair. The shaving device may include several sensors configured to measure data relating to the device usage, such as a force sensor to measure how hard the person is pressing the shaver against their face, and an inertial measurement unit (IMU) to measure parameters relating to the motion of the shaving device (e.g. direction of motion and speed of motion). While the person uses the shaving device to shave their facial hair, data is acquired by the force sensor and the IMU. Other sensors, such as a light sensor and/or an imaging device, may be provided on the shaving device itself or external to the shaving device, and these may measure data indicative of the outcome of the shaving activity. For example, the imaging device may provide an indication of the closeness of the shave, and the light sensor may detect signs of skin irritation (e.g. redness) resulting from the shaving activity. The device usage data is then compared with reference data relating to the usage of the device, and the outcome data is compared to reference outcome data relating to a shaving activity. If the measured data differs from the respective reference data by at least a threshold amount, then the user of the device may be asked to provide input. For example, the user may be asked to confirm whether they were aware that they were pressing the shaving device particularly forcefully on their skin and/or whether they feel any discomfort on their face following the shaving activity. If the user's input concurs with the measurements made using the sensors, then feedback may be provided to the user to suggest a change that could improve the performance of the shaving activity in future. However, if the user's input conflicts with the measurements made using the sensors, then it may be determined that one or more of the sensor sensitivities should be adjusted or that a model used to analyse the measured data should be optimised. Thus, the input provided by the user can be used to make adjustments or optimisations associated with the shaving device.

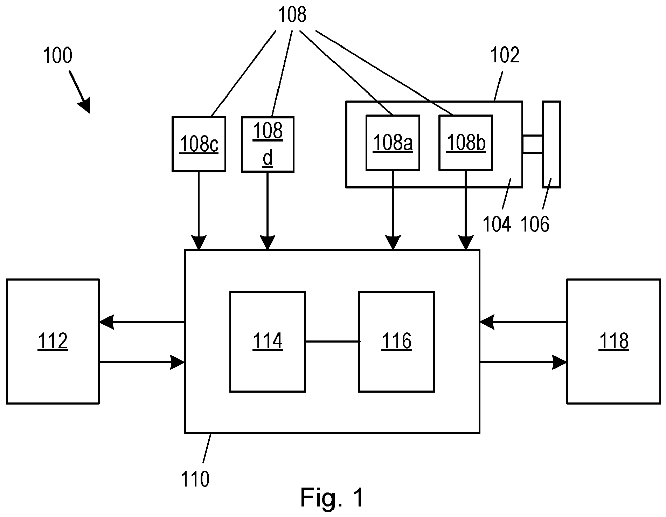

Embodiments of the invention will now be described with reference to the drawings. Fig. 1 is a schematic illustration of an example of a system 100 for interacting with a user of a personal care device 102. The personal care device 102 may comprise any type of personal care device used to perform a personal care activity. Some embodiments are described in the context of a handheld shaving device used to shave or trim hair from the skin of a subject. However, it will be appreciated that the invention is not limited to shaving devices, and embodiments described herein may be implemented in a wide range of personal care devices. Thus, the personal care device 102 described herein may, for example, comprise a hair removal device, such as an epilator or an intense pulsed light (IPL) device, a hair care device, such as a shaver, clippers or a hair trimmer, an oral care device, such as a power toothbrush or a flossing device, a skin health analysis device, an electric massager, a phototherapy device or a pain relief device. Other personal care devices are also envisaged.

The personal care device 102 may be intended to be held by a user's hand during use and, as such, may be referred to as a handheld personal care device. The personal care device 102 may comprise a body 104 and a treatment element 106 for performing the treatment. For example, the treatment element 106 of a shaving device may comprise a shaving head or cutting element, while the treatment element of a power toothbrush may comprise the brush head. The body 104 may house one or more components of the personal care device 102. In the example shown in Fig. 1, the body 104 comprises sensors 108a and 108b. While, in this example, the personal care device 102 is shown to include two sensors 108, in other examples, more or fewer sensors may be included. Furthermore, while the sensors 108 are shown in Fig. 1 to be housed within the body 104 of the personal care device 102, in other examples, one or more sensors may be located on or within any part of the personal care device, such as on or in the treatment element 106 or on a surface of the body 104.

Each of the sensors 108 may comprise any sensor capable of measuring data relating to the usage of the personal care device 102, referred to herein as device usage data. For example, each of the sensors 108 may comprise any sensor capable of measuring motion of the personal care device 102, such as a displacement of the personal care device during a usage instance of the personal care device. As used herein, the term "usage instance" is intended to refer to a session during which a user uses the personal care device 102 (e.g. to shave their facial hair or to brush their teeth). One or more of the sensors 108 may comprise an inertial measurement unit (IMU). An IMU, which will be familiar to those skilled in the art, may use an accelerometer, a gyroscope and/or a magnetometer to measure various parameters (e.g. specific force, angular rate and/or orientation) of a device in which the IMU is installed. An IMU may be used to measure several parameters of the personal care device 102 during a usage instance including, for example, a direction of movement of the personal care device, a speed of movement and, for a shaving device, an indication of whether the motion is with or against the direction of the user's facial hair (sometimes referred to as the grain direction). Data from such a sensor may be used to determine whether or not the user is moving the personal care device 102 at an appropriate speed and in an appropriate manner.

Data indicative of the speed of motion and/or a direction of motion of the personal care device 102 may also be measured using a sensor in a wearable device, such as a smartwatch, or calculated from data acquired by an imaging device, such as a camera.

One or more of the sensors 108 may comprise a force sensor or a pressure sensor to measure a force or pressure being applied by the personal care device 102 onto a surface, such as the skin of the user. For example, a force sensor a measure a force being applied between the treatment element 106 (e.g. a shaving head) and the user's skin. Various force sensors will be familiar to those skilled in the art, including switch-type sensors and thin-film resistive sensors. Generally, however, any suitable force sensor may be used. Data from such a force sensor may be used to determine whether or not the user is applying appropriate force onto their skin with the personal care device 102.

One or more of the sensors 108 may comprise a capacitive sensor. A capacitive sensor may be used, for example, for measuring and amount of the treatment element 106 that remains in contact with a surface (e.g. the user's skin or tooth) during the usage instance of the personal care device 102. Data from such a capacitive sensor may be used to determine whether or not the user is holding the personal care device 102 in an appropriate manner during use. A capacitive sensor may also be used for making skin hydration measurements, for measuring facial hair properties, such as heir length, hair density, and the like, and for measuring a force applied by the device on a surface, such as the user's skin.

In addition to the sensors 108a and 108b located about the personal care device 102, one or more additional sensors may be used to acquire data in respect of a usage instance of the personal care device. For example, the system 100 of Fig. 1 includes additional sensors 108c and 108d which are external to the personal care device 102 but which may be used to measure data relating to the use of the personal care device. One or more of the sensors 108 (e.g. the additional sensors 108c, 108d) may comprise an image capture device, such as a camera, for capturing images or video footage of a usage instance where the personal care device 102 is used to perform a personal care activity. One or more of the sensors 108 may comprise a context sensor to acquire data indicative of a context of the user and/or of the personal care device 102 during the usage instance. Example, such a context sensor may acquire data relating to the user's location, the weather (e.g. temperature and/or humidity) in the vicinity of the user, and/or the surroundings of the user (e.g. with user is located indoors or outdoors). Data from such a context sensor may be used in subsequent adjustments and optimisations of the sensors and/or of a model used to determine when to prompt the user to provide an input. While the sensors 108c and 108d are described herein as being external to the personal care device 102, in some examples, one or more of these additional sensors may be located in or on the personal care device. In other examples, one or more of the sensors 108 may be located in or on, or may form part of, another device, such as a smartphone, a wearable device, a tablet computer, a laptop computer, or an interactive mirror.

Each of the sensors 108 may communicate (e.g. via a wired or wireless connection) with a processor 110. The processor 110 may form part of an apparatus, for example a computing device such as a desktop computer, laptop computer, a tablet computer, a smart phone, wearable device, an interactive mirror or a server. In some examples, the processor 110 and one or more of the sensors 108 may form part of or be included in the same apparatus or device. The processor 110 may be connected (e.g. via a wired or wireless connection) to, or may otherwise communicate with, a storage device 112, such as a memory. In some examples, the storage device 112 and the processor 110 may be located within the same apparatus or device. The storage device 112 may be configured to receive and store data from the processor 110, such as data acquired using one or more of the sensors 108. The storage device 112 may also be configured to store reference data which can be used by the processor 110 for comparison with data acquired using one or more of the sensors 108.

According to some embodiments, the processor 110 may comprise or utilise a comparison module 114 and an analysis module 116. The functions of the processor 110 are discussed in greater detail below. In general, however, the processor 110 (e.g. the comparison module 114) compares data received from one or more of the sensors 108 with reference data stored in the storage device 112, and detects any significant deviation in the measured data from the referenced data. Specifically, the processor 110 or comparison module 114 determines whether the measured data differs from the reference data by more than a defined threshold amount. If a deviation from the reference data is detected or identified, then the processor 110 may cause a request for user input to be generated.

The reference data stored in the storage device 112 may, in some embodiments, comprise data that is to be expected with "normal" or "optimal" use of the personal care device 102. For example, the referenced data may represent data that would be obtained in respect of the personal care device 102 in an ideal situation, if the device were used during a usage instance in the way that the manufacturer intended. For a shaving device, for example, the referenced data may include an "ideal" speed of movement of the shaving device over the user's skin, and an "ideal" force with which the shaving device should be applied to the user's skin. In other embodiments, the reference data may comprise historical data acquired in respect of the personal care device 102 and/or of the user. For example, data acquired during one or more usage instances of the personal care device 102 by the user may be recorded and stored in the storage device 112, and this historical data may be used as the reference data. In some examples, the reference data may comprise an average of the historical data acquired over multiple usage instances and/or over a defined time period (e.g. a week, a month, or the like). In this way, data acquired during each new usage instance can be compared against data representing the user's average performance, and the processor 110 can detect if the user is using the device differently to normal. In some embodiments, the measured data may be compared with two sets of reference data: a first set of reference data representing an "optimal" usage of the personal care device 102, and a second set of reference data representing historical usage data of the personal care device/user.

A deviation from the reference data may be caused for several reasons. As noted above, a deviation may result simply from the user operating the personal care device 102 in a different way from usual (e.g. from their average usage), or in a manner that differs from the optimal usage. In other examples, a deviation may be detected because the threshold values against which the comparison is measured are inappropriate, or need to be changed. A change in the environment or context of the user may also lead to a deviation from the referenced data. For example, if the user were to visit a different location, with an increased humidity, then this may affect the ability of the user to perform a shaving activity as effectively and may, for example, lead to increased skin irritation compared to a less humid environment.

The request for user input may be provided to a user via a user interface 118. The user interface 118 may comprise one more of a display, a touchscreen, a speaker, or any other device capable of presenting information to the user. In some embodiments, the user interface 118 may further comprise a mechanism by which a user can input information. For example, the user interface 118 may comprise a touchscreen, a keyboard, a microphone or any other device capable of receiving information from the user.

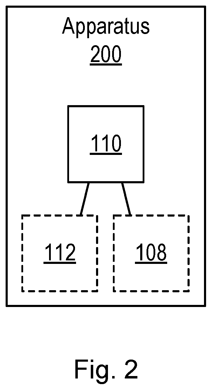

According to an aspect, an apparatus is provided. Fig. 2 is a schematic illustration of an example of an apparatus 200. The apparatus 200 may be considered to be an apparatus for interacting with a user of a personal care device, such as the personal care device 102. The apparatus 200 comprises a processor, such as the processor 110 discussed herein. The processor 110 is configured to receive device usage data indicative of a manner in which the personal care device is used by the user during a usage instance of the personal care device, the device usage data relating to one or more of a defined set of device usage parameters. The device usage data may be acquired using one or more of the sensors 108 discussed above, including sensors located on or within the personal care device 102 and/or sensors located externally with respect to the personal care device. Each device usage parameter to which the device usage data relates may comprise one of the following: a motion of the personal care device 102; a pressure applied by the personal care device onto a surface of the body of the user; a speed of movement of the personal care device; and a direction of motion of the personal care device. Motion, speed of movement and direction of motion of the personal care device 102 may, in some embodiments, be measured using an accelerometer, a gyroscope, a magnetometer and/or a component such as an IMU which includes a combination of sensors for measuring these parameters. A pressure applied by the personal care device 102 onto a surface of the body of the user may, in some embodiments, be measured using a pressure sensor or a force sensor, as described above. In some embodiments, data relating to one or more of the device usage parameters may be measured using another sensor 108, such as a camera. In some embodiments, data may be acquired using a combination of sensors 108. For example, the direction of motion of a shaving device may be measured in order to determine whether or not a user is moving the shaving device with, or against, the grain direction (i.e. the growth direction) of their facial hair. The grain direction may be determined using data acquired by a camera, and the direction of motion of the shaving device may be acquired using an IMU located within the shaving device.

The processor 110 is also configured to receive performance data indicative of an outcome of the usage instance, the performance data relating to one or more of a defined set of performance parameters. The performance data may be acquired using one or more of the sensors 108 discussed above, including sensors located on or within the personal care device 102 and/or sensors located externally with respect to the personal care device. Each performance parameter to which the performance data relates may comprise a parameter relating to an outcome of the personal care activity being performed. For example, when an oral care activity is being performed, such as toothbrushing, performance parameters may include an indication of gum damage or redness, an indication of bleeding from the gums, an indication of dirt/debris remaining on a tooth, and the like. As noted above, in some embodiments, the personal care device 102 may comprise a shaving device. In such examples, each outcome parameter may comprise one of the following: a closeness of shave achieved using the shaving device; an indication of skin irritation resulting from the usage instance; and an indication of redness of skin resulting from the usage instance.

The processor 110 is configured to generate a user input request to be presented to the user, responsive to a determination that one or more of the received device usage data and the received performance data differ from stored reference device usage data and stored reference performance data, respectively, by more than a defined threshold amount. Thus, the processor 110 may be configured to compare the received device usage data with stored reference device usage data and may compare the received performance data with stored reference performance data. If either comparison results in a difference by more than the defined threshold, the user input request is generated. For example, the processor 110 may generate a user input request if it determines that the received device usage data (e.g. a speed of movement of the shaving device over the face of the user) differs from (e.g. is faster or slower than) stored reference device usage data (e.g. a stored reference speed of movement indicating an average speed of movement of the shaving device by the user during past usage instances) by more than a defined threshold amount (e.g. by more than +/- 20 cm/s, or +/- 10%). In another example, the processor 110 may generate a user input request if it determines that the received performance data (e.g. an indication of the redness of the user's skin following a usage instance) differs from (e.g. is redder than) stored reference performance data (e.g. an indication of the average redness of the user's skin following past usage instances) by more than a defined threshold amount (e.g. by more than a defined amount of redness indicated, for example, using a color chart or a colorimeter). A threshold value, amount or range may be defined for each type of device usage data and performance data. Thus, the received device usage data may be compared to stored reference device usage data relative to a device usage threshold, and the received performance data may be compared to stored reference performance data relative to a performance threshold. In some examples, the processor 110 may generate guidance to be presented to the user, for example if the received device usage data deviates from an average of historic data.

The comparison of the received data with the stored reference data may, in some examples, be performed using the comparison module 114 described above. The analysis module 116 may be considered to perform more processing than a mere comparison data; for example, the analysis module may analyse received data and the stored reference data to detect potential errors in the received data (i.e. indicative of a fault with one or more of the sensors 108), and/or to detect potential errors in a model used by the comparison module 114 or the analysis module. For example, such a model may receive as its inputs the device usage data and the performance data, and the model may be configured to analyse the data in a particular way (e.g. using different weights for different types of data, and/or relying more heavily on some types of data in particular conditions than other types of data). Such a model may also use algorithms, logic or a rule-based process to determine what to ask the user (e.g. what type of user input to provide).

When the processor 110 detects a deviation in the received data from the stored reference data, it generates a request for user input from a user. The request for user information, for example, be provided to the user via the user interface 118. In some embodiments, the user input request may comprise a request for confirmation of one or more of the received device usage data and the received performance data. For example, if the received device usage data indicates that a user moved a shaving device particularly quickly over their face, then the user input request may comprise a request for confirmation that the user did indeed move the shaving device quicker than normal. If the user provides a response confirming the faster than usual movement of the shaving device, then the user may be provided with advice to improve their shaving device usage, for example a recommendation to move the shaving device slower. However, if the user provides a response denying that they moved the shaving device faster than usual, then the processor 110 may determine that one or more of the sensors 108 has a suspected fault, or that a sensor setting should be modified.

In some embodiments, the user input request may comprise a request for an indication of the user's perceived outcome of the usage instance relating to one or more of a defined set of perceived performance parameters. For example, it may be helpful to receive an indication from the user of how they feel the personal care activity went including, for example, an indication of the user's view on the outcome of the personal care activity. In examples where an oral care activity is being performed, such as toothbrushing, the user may be asked to provide an indication of their perceived outcome of the oral care activity based on perceived performance parameters such as an indication of whether or not their gums hurt, an indication of whether or not they have experienced bleeding from the gums, an indication of how clean their teeth feel, and the like. In examples where the personal care device 102 comprises a shaving device, and the user uses the shaving device to perform a shaving activity, each perceived performance parameter may comprise one: a perceived closeness of shave achieved using the shaving device; an indication of any perceived skin irritation resulting from the usage instance; and an indication of perceived comfort following the usage instance. Thus, in some examples, the perceived performance parameters may correspond to the received/measured performance parameters.

In response to providing a request for user input, the user may provide input using the user interface 118. In some examples, the user may type or speak an answer into the user interface. In other examples, the user may be presented with a list of options, and may provide their answer by selecting one or more of the presented options. For example, the user may be asked to indicate whether they feel that they applied more pressure on their face with the shaving device than usual, the same amount as pressure as normal, or less pressure than normal. Based on the response (i.e. the user input) provided by the user, the processor 110 may take an action, such as making an adjustment to parameters of the model, or to the defined thresholds. Thus, the processor 110 may, in some embodiments, be further configured to receive a user input relating to the usage instance of the personal care device 102; and adjust the defined threshold amount according to the received user input. For example, if it is determined that a measured parameter (e.g. speed of motion of a shaving device) regularly differs from the user's average speed of motion, but the input provided by the user indicates that they do not feel that they are moving the device any differently from normal, then it may be determined that a sensitivity ought to be changed. Accordingly, the defined threshold amount for that parameter may be adjusted (e.g. increased).

Some situations occurring which result in the processor 110 requesting input from the user may be referred to as faults, or faulty outcomes. Specifically, such situations may be considered to result from a fault in the model used by the processor 110 to analyse the data, or when the model provides an output (e.g. an indication that the user is pressing too hard on their face with the personal care device 102) that is different than expected. In some embodiments, the model used by the processor 110 (or the analysis module 116) may provide outputs with a level of confidence, or with an associated probability of the output. If the confidence of an output, or the probability of an output, is low, then the user may be asked to provide an input in order to increase the confidence level of the output of the model or, conversely, to lead to a change to the model in order to increase the confidence level of future outputs. As noted above, the user may be requested to provide input when received data indicates that the user's behaviour (e.g. from the device usage data) deviates from routine (e.g. from an average calculated from the stored reference data). In some examples, anomaly detection techniques may be used to detect deviations.

In some embodiments, a user input may be requested when the device usage data varies during a usage instance. For example, if it is detected that a user brushes their teeth in one side of their mouth differently from the way in which they brush the teeth in the other side of their mouth, or if they shave one side of the face differently from the other, then a fault may be suspected, and the user may be asked to provide an input. Thus, the stored data may comprise data relating to the same usage instance.

In another embodiment, a fault may be suspected if the user's environment changes (e.g. if the user is in a different location, such as during a vacation). Environmental changes may be determined using context data. Thus, in some embodiments, the processor 110 may be configured to receive context data indicative of the environment of the user of the personal care device 102 during the usage instance. Context data may be acquired using one or more of the sensors 108. In some embodiments, context data may include an indication of a geographical location, a temperature, an indication of the local weather, a humidity, or the like. The processor 110 may be configured to generate a user input request responsive to determining that the context data differs from stored reference context data by more than a defined threshold amount. In some embodiments, reference context data, such as data indicative of the usual environment in which the user performs personal care activities, may be stored in the storage device 112. The reference context data may, for example, comprise context data acquired during previous device usage instances.

As discussed above, the user may also be requested to provide input when it is detected that there is room for improvement in the performance of a personal care activity. In some embodiments, it may be determined that there is room for improvement by comparing the received usage data with reference data indicative of a preferred performance (e.g. a performance intended by the manufacturer of the personal care device or a performance preferred by the user), stored, for example, in the storage device 112. For example, a user may specify a preferred closeness of shave prior to commencing a shaving activity and, upon detecting a difference between data indicative of the closeness of shave measured during a usage instance and data indicative of the user's preferred closeness of shave, a delta may be computed. The delta may be considered an indication of the possible improvement available to the user.

In some embodiments, the storage device 112 may be located remotely with respect to the apparatus 200, and the processor 110 of the apparatus may access the storage device via a network connection. In other embodiments, however, the apparatus 200 may comprise a storage device, such as the storage device 112. The processor 110 may be configured to store one or more of the received device usage data and the received performance data in the storage device 112. In this way, the processor 110 may access the storage device 112 locally. The apparatus 200 may, in some embodiments, further comprise one or more sensors (e.g. the sensors 108) configured to acquire the device usage data and/or the performance data.

Various example use cases of the apparatus 200 are discussed below.

### Example 1

In a first example use case, a user performs a personal care activity using a shaving device. The sensors 108 measure device usage data indicating that the user moved the shaving device faster than the average speed of movement in the previous week. The sensors 108 also measure performance data indicating that the closeness of shave has decreased compared to the average closeness measured during the previous week. The comparison with the previously-recorded data may be performed using the comparison module 114 of the processor 110, and the analysis module 116 of the processor may perform further analysis to determine an action to be taken (e.g. a message to be communicated to the user). Analysis, in some embodiments, may be performed using a rule-based process while, in other embodiments, analysis may be performed using a machine learning algorithm. In this example, a rule-based database may be used to accommodate the increase in the speed of movement of the shaving device and the decrease in the closeness of shave. In one example, such a rule in the rule-based database may read:
*'if [correlation between clean shave and speed is found] then output guidance 'You have been moving [average delta in speed] faster than the last week; however, your clean shave result has decreased [average delta in clean shave]. Try to move slower. '*

Thus, according to one example, the following guidance may be provided to the user:
*'You have been moving 50%faster than the previous week; however, your clean shave result has decreased 30%. Try to move slower. '*

Alternatively, the user may be presented with a request to evaluate their shave by providing their view on how clean the shave was, for example on a continuous scale. In response to the user's input, a threshold and/or a setting of a sensor used to measure the closeness of the shave may be adjusted.

### Example 2

In a second example use case, in which a user performs a personal care activity using a shaving device, the sensors 108 measure device usage data indicating that the user has applied significantly more pressure on the left side of their chin compared to the right side of their chin. The sensors 108 also measure performance data indicating a slight increase in skin irritation on the left side of the user's chin. Again, the comparison with the previously recorded data may be performed using the comparison module 114 of the processor 110. The model used by the processor 110 (e.g. by the analysis module 116) is not entirely confident that there is a correlation between the increased pressure and the increased skin irritation and, therefore, it may be determined that user input should be requested. Thus, the processor 110 generates a prompt to be provided to the user:
*'You applied more pressure on the left side of your chin. Do you feel irritation on the left side of your left chin after shaving?*
*a) I do feel irritation*
*b) I feel a little irritation*
*c) I do not feel any irritation at all.*'

Depending on the answer provided by the user, the processor 110 may update the threshold when to request user input and/or may adjust a sensitivity of one or more of the sensors 108. In examples where a machine learning algorithm (or some other predictive model or artificial intelligence technique) is used for the analysis, one or more parameters (e.g. weights) of the algorithm may be adapted. If the user were to answer *'c) I do not feel any irritation at all',* then the processor 110 may arrange for the ground truth used in the model (or used by the analysis module 116) to be overwritten and/or arrange for some method of updating the model to be performed, such as a backpropagation. If, however, the user were to answer *'a) I do feel irritation'*, then no update of the model is required. Instead, the user may be provided with a recommendation to vary the shaving technique (e.g. reduce the pressure applied) when shaving the left side of the chin.

### Example 3

In a third example use case, a user is again performing a personal care activity using a shaving device. In this example, the sensors 108 measure device usage data indicating that the user is applying significantly less pressure on their face compared to the pressure applied during historical usage instances for that user. The processor 110 generates a query to be provided to the user, that reads:

### Are you shaving?'

If the user provides an input indicating that they are not shaving, then this confirms the determination made based on the measurements. However, if the user's input indicates that they are shaving, then parameters of the model may be updated so that a measurement of a similar pressure in the future is recognised as corresponding to a usage instance of the shaving device.

### Example 4

In a fourth example, in which the user is again performing a personal care activity using shaving device, the sensors 108 measure device usage data indicating that significantly more pressure is being applied by the shaving device on the user's face, and the sensors measure performance data that do not indicate any signs of skin irritation. The processor 110 generates a query to be provided to the user, that reads:
*'It looks like you are applying more pressure than usual; do you feel any skin irritation?'*

If the user responds with *'Yes'*, then the processor 110 may arrange for a setting and/or a threshold to be adjusted in respect of one or more of the sensors 108 (e.g. the sensor measuring the applied pressure and/or the sensor measuring apparent skin irritation). If, however, the user responds with *'No'*, then parameters of the model may be updated so that a measurement of a similar pressure in the future does not result in a suspected skin irritation outcome.

While it is clear from the above discussions that a determination to request a user input may be made if one or more of the measured data exceeds a corresponding reference data by more than a defined threshold, embodiments of the present invention also enable a determination to be made regarding what input is to be requested from the user (e.g. what to ask the user). For example, if the sensors 108 detect differences in multiple measured parameters with respect to corresponding reference data, then a decision is made which parameter is to be the subject of the query to the user. For example, sensors 108 may be used to measure pressure, speed of movement and skin irritation in respect of a usage instance of a shaving device. Table 1 below shows examples of three different usage instances, during which measured data may indicate that each parameter is more/greater than usual (+), the same as usual (0) or lower/less than usual (-).

**Table 1**

| | Instance 1 | Instance 2 | Instance 3 |
|---|---|---|---|
| Pressure | + | + | - |
| Speed | 0 | 0 | + |
| Skin irritation | 0 | - | + |
| *Candidate measurement for input* | Pressure | Skin irritation | Speed |

In usage instance 1, the measured data indicates that the speed of movement and the signs of skin irritation are the same as usual, despite an increase in pressure. In this example, therefore, the user may be asked to provide input regarding the pressure applied (e.g. by asking the user whether they did indeed apply greater pressure and/or whether the user experience the different feeling), so that this measurement can be confirmed, and appropriate action (e.g. changing parameters of the model or changing a sensitivity of the sensor) can be taken.

In usage instance 2, the measured data indicates that the speed of movement is the same as usual, but the signs of skin irritation are lower than usual, despite an increase in pressure. Since this outcome is surprising, the user may be asked to provide input regarding the skin irritation measurement, for example to check whether the skin irritation sensor is working.

In usage instance 3, the measured data indicates that the pressure applied by the user is the same as usual, but the speed of movement and the signs of skin rotation are both greater than usual. In this example, the user may be asked to provide input regarding the speed of movement of the personal care device 102, for example to check whether the user did actually increase the speed of movement of the device, in order to check whether or not this is related to the increased scan irritation.

In other examples, candidate measurements to form the basis of user input requests may be selected based, for example, on statistical parameters such as standard deviation/measurement error (e.g. the larger the standard deviation/measurement error, the greater the likelihood that the measurement should be selected), or on a knowledge of cause and effect (e.g. an increased pressure may cause greater skin irritation) so that the effect can be confirmed by the user.

According to another aspect, a system is provided. Examples of such a system 100 are shown in Fig. 1, as discussed above. The system 100 comprises one or more sensors 108 for acquiring device usage data and performance data. The device usage data is indicative of a manner in which a personal care device 102 is used by a user during a usage instance of the personal care device. The performance data is indicative of an outcome of the usage instance. The system 100 also comprises a storage module (e.g. the storage device 112) for storing reference device usage data and reference performance data. As described above, the reference data may comprise data indicative of an intended use/performance of the personal care device 102 and/or data indicative of an average use/performance of the personal care device by the user during previous usage instances (e.g. during a defined time period). The system 100 also comprises a comparison module 114 for comparing the acquired device usage data with the stored reference device usage data and for comparing the acquired performance data with the stored performance data. The system 100 also comprises a user interface 118 for presenting a user input request to a user. The user input request may be presented responsive to a determination that the acquired device usage data differs from the stored reference device usage data by more than a first defined threshold amount; or the acquired performance data differs from the stored reference performance data by more than a second defined threshold amount. The first and second defined threshold amounts may comprise absolute values, relative values, percentages or ranges.

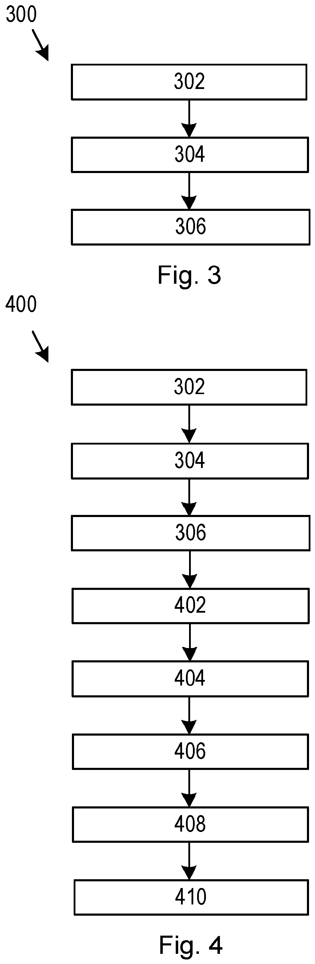

According to another aspect, a method is provided. Fig. 3 is a flowchart of an example of a method 300, such as a method for interacting with a user of a personal care device 102. The method 300 may comprise a computer-implemented method. The method 300 comprises, at step 302, receiving device usage data indicative of a manner in which the personal care device 102 is used by the user during a usage instance of the personal care device, the device usage data relating to one or more of a defined set of device usage parameters. At step 304, the method 300 comprises receiving performance data indicative of an outcome of the usage instance, the performance data relating to one or more of a defined set of performance parameters. The device usage data and/or the performance data may be received from one or more of the sensors 108 discussed herein. The method 300 comprises, at step 306, responsive to a determination that one or more of the received usage data and the received performance data differ from stored reference device usage data and stored reference performance data, respectively, by more than a defined threshold amount, generating a user input request to be presented to the user.

Fig. 4 is a flowchart of a further example of a method 400, such as a method for interacting with a user of a personal care device 102. The method 400 may also comprise a computer-implemented method, and may comprise one or more steps of the method 300 discussed above.

The user input request generated at step 306 may be presented to the user via the user interface 118. According to various embodiments, the user input request may comprise at least one of: a request for confirmation of one or more of the received device usage data and the received performance data; and a request for an indication of the user's perceived outcome of the usage instance relating to one or more of a defined set of perceived performance parameters. In some examples, the method may comprise generating guidance (e.g. a suggestion) to be presented to the user, for example if the received device usage data deviates from an average of historic data.

The user may provide input in response to the user input request via the user interface 118 or via any other suitable input device. At step 402, the method 400 may comprise receiving a user input relating to the usage instance of the personal care device. For example, the received user input may confirm one or more of the received device usage data and the received performance data and/or the received user input may indicate the user is perceived outcome of the usage instance. The method 400 may further comprise, at step 404, adjusting the defined threshold amount according to the received user input. For example, if it is determined, based on the user input, that the current threshold amount results in user input requests being generated too frequently, then threshold amount may be adjusted.

The user input request may comprise a request for confirmation of one or more of the received device usage data and the received performance data. In other examples, the user input request may comprise a request for an indication of the user's perceived outcome of the usage instance relating to one or more of a defined set of perceived performance parameters.

In examples where the personal care device is a shaving device, each perceived performance parameter comprises an outcome parameter selected from a group comprising: a perceived closeness of shave achieved using the shaving device; an indication of any perceived skin irritation resulting from the usage instance; and an indication of perceived comfort following the usage instance. Similarly, in examples where the personal care device is a shaving device, each outcome parameter may comprise an outcome parameter selected from a group comprising: a closeness of shave achieved using the shaving device; an indication of skin irritation resulting from the usage instance; and an indication of redness of skin resulting from the usage instance.

Each device usage parameter may comprise a usage parameter selected from a group comprising: a motion of the personal care device; a pressure applied by the personal care device onto a surface of the body of the user; a speed of movement of the personal care device; and a direction of motion of the personal care device.

In other examples, the method 300, 400 may comprise further steps corresponding to functions performed by various components (e.g. the processor 110) as discussed herein. For example, the method 400 may further comprise, at step 406, receiving context data indicative of the environment of the user of the personal care device during the usage instance. At step 408, the method 400 may further comprise generating a user input request responsive to determining that the context data differs from stored reference context data by more than a defined threshold amount.

The method 400 may comprise, at step 410, responsive to a determination that one or more of the received device usage data and the received performance data differ from stored reference device usage data and stored reference performance data, respectively, by more than a defined threshold amount, in respect of two or more device usage parameters or performance parameters, generating a user input request to be presented to the user, the user input request being in respect of the device usage parameter or performance parameter corresponding to the largest determined difference. For example, as shown in Table 1 above, if the received data differs from the stored data in respect of multiple parameters, then the user is asked to provide input in respect of the parameter having the largest difference, as this may be considered to be the most significant issue to address.

In some embodiments, at least one of the stored reference device usage data and the stored reference performance data comprises data relating to at least one previous device usage instance of the personal care device by the user. For example, data measured during each usage instance by the user may be stored and used as the reference data in comparison to data measured in future usage instances.

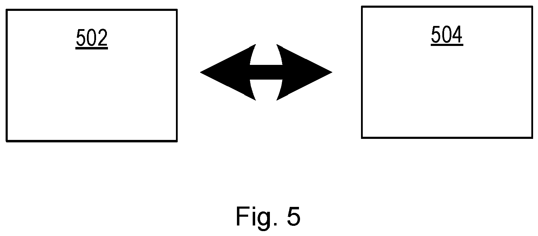

According to another aspect, a computer program product is provided. Fig. 5 is a schematic illustration of an example of a processor 502 in communication with a computer-readable medium 504. According to various embodiments, a computer program product comprises a non-transitory computer-readable medium 504, the computer-readable medium having computer-readable code embodied therein, the computer-readable code being configured such that, on execution by a suitable computer or processor 502, the computer or processor is caused to perform steps the methods 300, 400 disclosed herein. The processor 502 may comprise, or be similar to, the processor 110 discussed above.

The processor 110, 502 can comprise one or more processors, processing units, multi-core processors or modules that are configured or programmed to control the apparatus 200 in the manner described herein. In particular implementations, the processor 110, 502 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (300) for interacting with a user of a personal care device (102), the method comprising:
receiving (302) device usage data indicative of a manner in which the personal care device is used by the user during a usage instance of the personal care device, the device usage data relating to one or more of a defined set of device usage parameters;
receiving (304) performance data indicative of an outcome of the usage instance, the performance data relating to one or more of a defined set of performance parameters; and
responsive to a determination that one or more of the received device usage data and the received performance data differ from stored reference device usage data and stored reference performance data, respectively, by more than a defined threshold amount, generating (306) a user input request to be presented to the user.

2. A computer-implemented method (300) according to claim 1, further comprising:
receiving (402) a user input relating to the usage instance of the personal care device; and
adjusting (404) the defined threshold amount according to the received user input.

3. A computer-implemented method (300) according to claim 1 or claim 2, wherein the user input request comprises a request for confirmation of one or more of the received device usage data and the received performance data.

4. A computer-implemented method (300) according to any of the preceding claims, wherein the user input request comprises a request for an indication of the user's perceived outcome of the usage instance relating to one or more of a defined set of perceived performance parameters.

5. A computer-implemented method (300) according to claim 4, wherein the personal care device (102) comprises a shaving device; and
wherein each perceived performance parameter comprises an outcome parameter selected from a group comprising: a perceived closeness of shave achieved using the shaving device; an indication of any perceived skin irritation resulting from the usage instance; and an indication of perceived comfort following the usage instance.

6. A computer-implemented method (300) according to any of the preceding claims, wherein each device usage parameter comprises a usage parameter selected from a group comprising: a motion of the personal care device; a pressure applied by the personal care device onto a surface of the body of the user; a speed of movement of the personal care device; and a direction of motion of the personal care device.

7. A computer-implemented method (300) according to any of the preceding claims, wherein the personal care device (102) comprises a shaving device; and
wherein each outcome parameter comprises an outcome parameter selected from a group comprising: a closeness of shave achieved using the shaving device; an indication of skin irritation resulting from the usage instance; and an indication of redness of skin resulting from the usage instance.

8. A computer-implemented method (300) according to any of the preceding claims, further comprising:
receiving (406) context data indicative of the environment of the user of the personal care device during the usage instance; and
generating (408) a user input request responsive to determining that the context data differs from stored reference context data by more than a defined threshold amount.

9. A computer-implemented method (300) according to any of the preceding claims, further comprising:
responsive to a determination that one or more of the received device usage data and the received performance data differ from stored reference device usage data and stored reference performance data, respectively, by more than a defined threshold amount, in respect of two or more device usage parameters or performance parameters, generating a user input request to be presented to the user, the user input request being in respect of the device usage parameter or performance parameter corresponding to the largest determined difference.

10. A computer-implemented method (300) according to any of the preceding claims, wherein at least one of the stored reference device usage data and the stored reference performance data comprises data relating to at least one previous device usage instance of the personal care device by the user.

11. An apparatus (200) for interacting with a user of a personal care device (102), the apparatus comprising:
a processor (110) configured to perform steps of the methods of any of the preceding claims.

12. An apparatus (200) according to claim 11, further comprising:
a storage device (112);
wherein the processor is configured to store one or more of the received device usage data and the received performance data in the storage device.

13. An apparatus (200) according to claim 11 or claim 12, further comprising:
one or more sensors (108) configured to acquire the device usage data and/or the performance data.

14. A system (100) comprising:
one or more sensors (108) for acquiring:
device usage data indicative of a manner in which a personal care device is used by a user during a usage instance of the personal care device; and
performance data indicative of an outcome of the usage instance;
a storage module (112) for storing reference device usage data and reference performance data;
a comparison module (114) for comparing the acquired device usage data with the stored reference device usage data and for comparing the acquired performance data with the stored performance data; and
a user interface (118) for presenting a user input request responsive to a determination that:
the acquired device usage data differs from the stored reference device usage data by more than a first defined threshold amount; or
the acquired performance data differs from the stored reference performance data by more than a second defined threshold amount.

15. A computer program product comprising a non-transitory computer-readable medium (504), the computer-readable medium having computer-readable code embodied therein, the computer-readable code being configured such that, on execution by a suitable computer or processor (502), the computer or processor is caused to perform the method of any of claims 1 to 10.
